(19) Europäisches Patentamt
European Patent Office
Office européen des brevets

(11) Veröffentlichungsnummer: **0 317 947**
**A2**

(12) **EUROPÄISCHE PATENTANMELDUNG**

(21) Anmeldenummer: **88119396.5**

(22) Anmeldetag: **22.11.88**

(51) Int. Cl.4: **C07D 471/04 , C07D 487/04 , A01N 43/90 , //(C07D471/04, 249:00,221:00),(C07D487/04, 249:00,209:00),(C07D487/04, 249:00,223:00)**

(30) Priorität: **27.11.87 DE 3740837**

(43) Veröffentlichungstag der Anmeldung:
**31.05.89 Patentblatt 89/22**

(84) Benannte Vertragsstaaten:
**AT BE CH DE ES FR GB GR IT LI LU NL SE**

(71) Anmelder: **SCHERING AKTIENGESELLSCHAFT**
**Berlin und Bergkamen**
**Müllerstrasse 170/178 Postfach 65 03 11**
**D-1000 Berlin 65(DE)**

(72) Erfinder: **Blume, Friedhelm, Dr.**
**Nusshäherstrasse 47 T**
**D-1000 Berlin 27(DE)**
Erfinder: **Dorfmeister, Gabriele, Dr.**
**Heiligenseestrasse 70**
**D-1000 Berlin 27(DE)**
Erfinder: **Franke, Wilfried, Dr.**
**Spiessergasse 6 b**
**D-1000 Berlin 27(DE)**
Erfinder: **Rees, Richard, Dr.**
**Speerweg 8**
**D-1000 Berlin 28(DE)**
Erfinder: **Johann, Gerhard, Dr.**
**Hermsdorfer Damm 147**
**D-1000 Berlin 28(DE)**
Erfinder: **Arndt, Friedrich, Dr.**
**Mühlenfeldstrasse 10**
**D-1000 Berlin 28(DE)**

(54) **Substituierte bicyclische Triazole, Verfahren zu ihrer Herstellung und ihre Verwendung als herbizide Mittel.**

(57) Die Erfindung betrifft neue substituierte bicyclische Triazole der allgemeinen Formel I

EP 0 317 947 A2

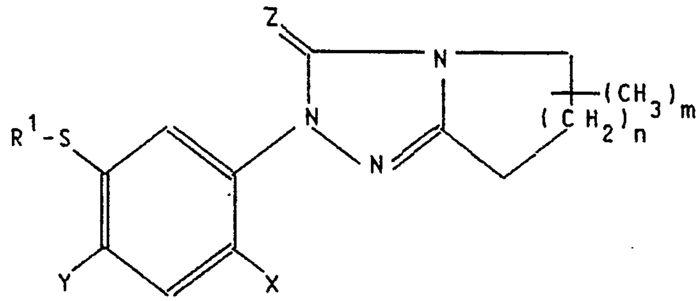

(I) ,

in der X, Y, Z, $R^1$, n und m die in der Beschreibung genannten Bedeutungen haben, Verfahren zu ihrer Herstellung und ihre Verwendung als herbizide Mittel.

# SUBSTITUIERTE BICYCLISCHE TRIAZOLE, VERFAHREN ZU IHRER HERSTELLUNG UND IHRE VERWENDUNG ALS HERBIZIDE MITTEL

Die Erfindung betrifft neue substituierte bicyclische Triazole, Verfahren zu ihrer Herstellung und ihre Verwendung als herbizide Mittel.

Es ist bereits bekannt, daß bestimmte bicyclische Triazole herbizide Eigenschaften besitzen (DE-OS 28 01 429). Bei diesen bekannten Verbindungen treten jedoch häufig Selektivitätsprobleme in wichtigen landwirtschaftlichen Kulturen auf.

Es wurde nun gefunden, daß substituierte bicyclische Triazole der allgemeinen Formel I

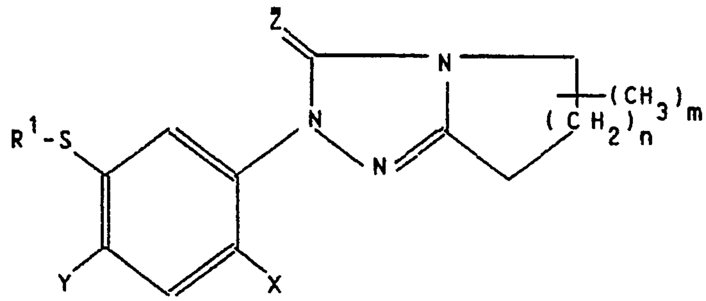

(I) ,

in der

X ein Wasserstoffatom oder ein Fluoratom,

Y ein Halogenatom,

Z ein Sauerstoffatom oder ein Schwefelatom,

$R^1$ einen $C_1$-$C_6$-Alkyl, $C_2$-$C_6$-Alkenyl- oder $C_3$-$C_6$-Alkinylrest, einen ein-oder mehrfach durch Halogen und/oder $C_1$-$C_4$-Alkoxy substituierten $C_1$-$C_6$-Alkyl-, $C_2$-$C_6$-Alkenyl- oder $C_3$-$C_6$-Alkinylrest, einen $C_3$-$C_7$-Cycloalkyl-oder $C_5$-$C_7$-Cycloalkenylrest, bei dem gegebenenfalls ein oder zwei Kohlenstoffatome durch Sauerstoffatome ersetzt sind, einen ein- oder mehrfach durch Halogen und/oder $C_1$-$C_4$-Alkoxy substituierten $C_3$-$C_7$-Cycloalkyl- oder $C_5$-$C_7$-Cycloalkenylrest, bei dem gegebenenfalls ein oder zwei Kohlenstoffatome durch Sauerstoffatome ersetzt sind, einen $C_3$-$C_7$-Cycloalkyl-$C_1$-$C_3$-alkyl- oder $C_5$-$C_7$-Cycloalkenyl-$C_1$-$C_3$-alkylrest, bei dem gegebenenfalls ein oder zwei Kohlenstoffatome durch Sauerstoffatome ersetzt sind, einen ein-oder mehrfach durch Halogen und/oder $C_1$-$C_4$-Alkoxy substituierten $C_3$-$C_7$-Cycloalkyl-$C_1$-$C_3$-alkyl-oder $C_5$-$C_7$-Cycloalkenyl-$C_1$-$C_3$-alkylrest, bei dem gegebenenfalls ein oder zwei Kohlenstoffatome durch Sauerstoffatome ersetzt sind, einen Cyano-$C_1$-$C_3$-alkylrest oder die Gruppe $CR^2R^3A$,

$R^2$ ein Wasserstoffatom, ein Fluoratom oder einen Methylrest,

$R^3$ ein Wasserstoffatom, ein Fluoratom oder einen Methylrest,

A eine der Gruppen $CO_2R^4$, $COSR^5$ oder $CONR^6R^7$,

$R^4$ einen $C_1$-$C_6$-Alkyl-, $C_2$-$C_6$-Alkenyl- oder $C_3$-$C_6$-Alkinylrest, einen ein-oder mehrfach durch Halogen und/oder $C_1$-$C_4$-Alkoxy substituierten $C_1$-$C_6$-Alkyl-, $C_2$-$C_6$-Alkenyl- oder $C_3$-$C_6$-Alkinylrest, einen $C_3$-$C_7$-Cycloalkyl-oder $C_5$-$C_7$-Cycloalkenylrest, bei dem gegebenenfalls ein oder zwei Kohlenstoffatome durch Sauerstoffatome ersetzt sind, einen ein- oder mehrfach durch Halogen und/oder $C_1$-$C_4$-Alkoxy substituierten $C_3$-$C_7$-Cycloalkyl- oder $C_5$-$C_7$-Cycloalkenylrest, bei dem gegebenenfalls ein oder zwei Kohlenstoffatome durch Sauerstoffatome ersetzt sind, einen $C_3$-$C_7$-Cycloalkyl-$C_1$-$C_3$-alkyl- oder $C_5$-$C_7$-Cycloalkenyl-$C_1$-$C_3$-alkylrest, bei dem gegebenenfalls ein oder zwei Kohlenstoffatome durch Sauerstoffatome ersetzt sind, einen ein-oder mehrfach durch Halogen und/oder $C_1$-$C_4$-Alkoxy substituierten $C_3$-$C_7$Cycloalkyl-$C_1$-$C_3$-alkyl-oder $C_5$-$C_7$-Cycloalkenyl-$C_1$-$C_3$-alkylrest, bei dem gegebenenfalls ein oder zwei Kohlenstoffatome durch Sauerstoffatome ersetzt sind, oder einen Cyano-$C_1$-$C_3$-alkylrest,

$R^5$ einen $C_1$-$C_6$-Alkyl-, $C_2$-$C_6$-Alkenyl- oder $C_3$-$C_6$-Alkinylrest oder einen ein- oder mehrfach durch Halogen und/oder $C_1$-$C_4$-Alkoxy substituierten $C_1$-$C_6$-Alkyl-, $C_2$-$C_6$-Alkenyl- oder $C_3$-$C_6$-Alkinylrest,

$R^6$ ein Wasserstoffatom, einen $C_1$-$C_6$-Alkyl-, $C_2$-$C_6$-Alkenyl- oder $C_3$-$C_6$-Alkinylrest oder einen Phenylrest,

$R^7$ ein Wasserstoffatom, einen $C_1$-$C_6$-Alkyl-, $C_2$-$C_6$-Alkenyl- oder $C_3$-$C_6$-Alkinylrest oder einen Phenylrest oder

$R^6$ und $R^7$ gemeinsam mit dem benachbarten Stickstoffatom die Morpholino-, Piperidino- oder Pyrrolidinylgruppe,

n die Zahlen 1, 2 oder 3 und

m die Zahlen 0, 1, 2 oder 3
bedeuten, eine sehr gute Verträglichkeit für Kulturpflanzen bei gleichzeitig interessanter herbizider Wirkung zeigen.

Der Ausdruck Halogen steht für Fluor, Chlor, Brom und Jod.

Die erfindungsgemäßen Verbindungen der allgemeinen Formel I lassen sich herstellen, indem man

A) falls Z für Sauerstoff steht, eine Verbindung der allgemeinen Formel II

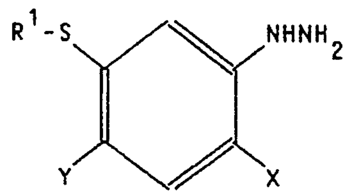

(II) ,

in der X, Y und $R^1$ die unter der allgemeinen Formel I genannten Bedeutungen haben, mit einer Verbindung der allgemeinen Formel III

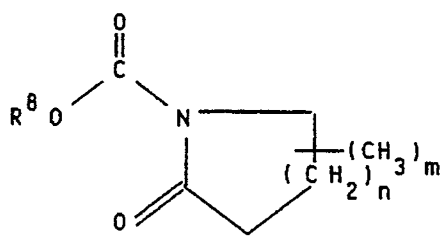

(III) ,

in der n und m die unter der allgemeinen Formel I genannte Bedeutung haben und $R^8$ für $C_1$-$C_4$-Alkyl steht, in einem inerten Lösungsmittel in Gegenwart von Phosphorpentoxid umsetzt, oder

B) eine Verbindung der allgemeinen Formel IV

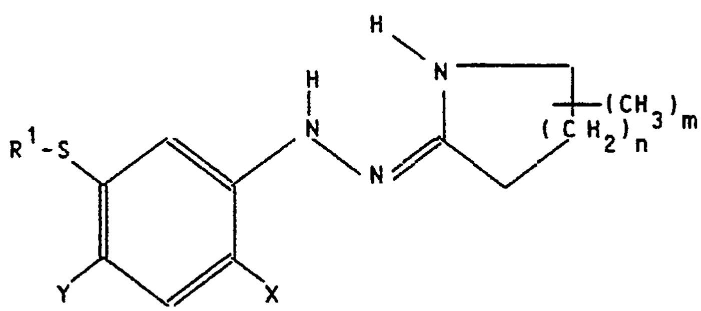

(IV) ,

in der X, Y, $R^1$, n und m die unter der allgemeinen Formel I genannten Bedeutungen haben, gegebenenfalls als Salz mit einer Verbindung der allgemeinen Formel V

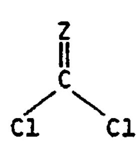

(V) ,

in der Z die unter der allgemeinen Formel I genannte Bedeutung hat, in einem inerten Lösungsmittel in Gegenwart einer Base umsetzt.

Die genannten Ausgangsmaterialien sind bekannt oder lassen sich analog zu bekannten Verfahren herstellen.

Die Verfahrensvariante A) wird zweckmäßigerweise so durchgeführt, daß man die Ausgangsmaterialien

der allgemeinen Formeln II und III in Gegenwart von Phosphorpentoxid in einem geeigneten Lösungsmittel bei Temperaturen zwischen 20 und 150 °C, vorzugsweise aber bei der Siedetemperatur des Lösungsmittels, zur Reaktion bringt. Als Lösungsmittel kommen Halogenkohlenwasserstoffe, wie zum Beispiel Methylenchlorid oder Chloroform, oder aromatische Kohlenwasserstoffe, wie zum Beispiel Benzol, Toluol, Xylol, Chlorbenzol oder Dichlorbenzol, in Frage. Die Reaktionszeit beträgt 0,5 bis 15 Stunden.

Die Verfahrensvariante B) wird zweckmäßigerweise so durchgeführt, daß man die Amidrazone der allgemeinen Formel IV oder ihre Salze mit Säuren (zum Beispiel Hydrochloride) entweder mit Phosgen (Z = O) oder Thiophosgen (Z = S) in einem geeigneten inerten Lösungsmittel in einem Temperaturbereich von 0 bis 150 °C, vorzugsweise aber bei der Siedetemperatur des Lösungsmittels, zur Reaktion bringt. Als Lösungsmittel kommen Halogenkohlenstoffe, wie zum Beispiel Methylenchlorid oder Chloroform, Ether, wie zum Beispiel Tetrahydrofuran oder Dioxan, oder aromatische Kohlenwasserstoffe, wie zum Beispiel Toluol, Xylol, Chlorbenzol oder Dichlorbenzol, in Frage. Zur Neutralisation der während der Reaktion freigesetzten Säure verwendet man eine geeignete Base, vorzugsweise ein Amin, wie zum Beispiel Pyridin oder Triethylamin. Die Reaktionszeit beträgt 1 bis 24 Stunden.

Die Aufarbeitung der nach den Verfahrensvarianten A) und B) hergestellten erfindungsgemäßen Verbindungen erfolgt in der üblichen Art und Weise. Eine Aufreinigung erfolgt durch Kristallisation oder Säulenchromatographie.

Die erfindungsgemäßen Verbindungen stellen in der Regel farblose oder schwach gelb gefärbte kristalline oder zähflüssige Substanzen dar, die zum Teil gut löslich in Ethern, wie zum Beispiel Tetrahydrofuran, Alkoholen, wie zum Beispiel Methanol oder Ethanol, halogenierten Kohlenwasserstoffen, wie zum Beispiel Methylenchlorid oder Chloroform, Sulfoxiden, wie zum Beispiel Dimethylsulfoxid, oder Estern, wie zum Beispiel Essigester, sind.

Die erfindungsgemäßen Verbindungen zeigen eine gute herbizide Wirkung bei breitblättrigen Unkräutern und Gräsern. Ein selektiver Einsatz der erfindungsgemäßen Wirkstoffe ist in verschiedenen Kulturen möglich, zum Beispiel in Raps, Rüben, Sojabohnen, Baumwolle, Reis, Gerste, Weizen und anderen Getreidearten. Dabei sind einzelne Wirkstoffe als Selektivherbizide in Rüben, Baumwolle, Soja und Getreide besonders geeignet. Ebenso können die Verbindungen zur Unkrautbekämpfung in Dauerkulturen, wie zum Beispiel Forst-, Ziergehölz, Obst-, Wein-, Citrus-, Nuß-, Bananen-, Kaffee-, Tee-, Gummi-, Ölpalm-, Kakao-, Beerenfrucht- und Hopfenanlagen, und zur selektiven Unkrautbekämpfung in einjährigen Kulturen eingesetzt werden.

Die erfindungsgemäßen Verbindungen können zum Beispiel bei den folgenden Pflanzengattungen verwendet werden:

Dikotyle Unkräuter der Gattungen Sinapis, Lepidium, Galium, Stellaria, Matricaria, Anthemis, Galinsoga, Chenopodium, Brassica, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Lamium, Veronica, Abutilon, Datura, Viola, Galeopsis, Papaver, Centaurea und Chrysanthemum.

Monokotyle Unkräuter der Gattungen Avena, Alopecurus, Echinochloa, Setaria, Panicum, Digitaria, Poa, Eleusine, Brachiaria, Lolium, Bromus, Cyperus, Agropyron, Sagittaria, Monochoria, Fimbristylis, Eleocharis, Ischaemum und Apera.

Die Aufwandmengen schwanken jenach Anwendungsart im Vor- und Nachauflauf in Grenzen zwischen 0,03 bis 5 kg/ha.

Die erfindungsgemäßen Wirkstoffe können auch als Defoliant, Desiccant und als Krautabtötungsmittel verwendet werden.

Die erfindungsgemäßen Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden. Sofern eine Verbreiterung des Wirkungsspektrums beabsichtigt ist, können auch andere Herbizide zugesetzt werden.

Beispielsweise eignen sich als herbizid wirksame Mischungspartner diejenigen Wirkstoffe, die in Weed Abstracts, Vol. 35, No.5, 1986, unter dem Titel "List of common names and abbreviations employed for currently used herbicides and plant growth regulators in Weed Abstracts" aufgeführt sind.

Eine Förderung der Wirkintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Zweckmäßig werden die gekennzeichneten Wirkstoffe oder deren Mischungen in Form von Zubereitungen, wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier-und/oder Dispergierhilfsmitteln angewandt.

Geeignete flüssige Trägerstoffe sind zum Beispiel aliphatische und aromatische Kohlenwasserstoffe,

wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Bentonit, Silicagel, Talkum, Kaolin, Attapulgit, Kalkstein, und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des beziehungsweise der Wirkstoffe(s) in den verschiedenen Zubereitungen kann in weiten Grenzen variieren. Beispielsweise enthalten die Mittel etwa 10 bis 90 Gewichtsprozent Wirkstoff, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen etwa 100 bis 1000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, zum Beispiel durch Mahl- oder Mischverfahren, durchgeführt werden. Gewünschtenfalls können Zubereitungen der Einzelkomponenten auch erst kurz vor ihrer Verwendung gemischt werden, wie es zum beispiel im sogenannten Tankmixverfahren in der Praxis durchgeführt wird.

Zur Herstellung der verschiedenen Zubereitungen werden zum Beispiel die folgenden Bestandteile eingesetzt:

A) **Spritzpulver**

1.) 20 Gewichtsprozent Wirkstoff
68 Gewichtsprozent Kaolin
10 Gewichtsprozent Calciumsalz der Ligninsulfonsäure
2 Gewichtsprozent Dialkylnaphthalinsulfonat

2.) 40 Gewichtsprozent Wirkstoff
25 Gewichtsprozent Kaolin
25 Gewichtsprozent kolloidale Kieselsäure
8 Gewichtsprozent Calciumsalz der Ligninsulfonsäure
2 Gewichtsprozent Natriumsalz des N-Methyl-N-oleyl-taurins

B) **Paste**

45 Gewichtsprozent Wirkstoff
5 Gewichtsprozent Natriumaluminiumsilikat
15 Gewichtsprozent Cetylpolyglycolether mit 8 Mol Ethylenoxid
2 Gewichtsprozent Spindelöl
10 Gewichtsprozent Polyethylenglycol
23 Gewichtsprozent Wasser

C) **Emulsionskonzentrat**

20 Gewichtsprozent Wirkstoff
75 Gewichtsprozent Isophoron
2 Gewichtsprozent ethoxyliertes Rizinusöl
3 Gewichtsprozent Calciumsalz der Dodecylphenylsulfonsäure

Die nachfolgenden Beispiele erläutern die Herstellung der erfindungsgemäßen Verbindungen:

**Beispiel 1**

2-(4-Chlor-5-ethoxycarbonylmethylthio-2-fluorphenyl)-5,6,7,8-tetrahydro-1,2,4-triazolo[4,3-a]pyridin-3(2H)-on

Eine Lösung von 4,2 g 4-Chlor-5-ethoxycarbonylmethylthio-2-fluorphenylhydrazin und 2,6 g N-Ethoxycarbonyl-2-piperidon in 50 ml Xylol wird mit 1,1 g Phosphorpentoxid versetzt und 3 Stunden unter Rückfluß erhitzt. Die Lösung wird mit Wasser versetzt und mit Essigester extrahiert. Die organische Phase wird über Magnesiumsulfat getrocknet, eingeengt und der Rückstand durch Säulenchromatographie gereinigt (Kieselgel, Elutionsmittel: Hexan/Essigester).
Ausbeute: 1,2 g = 21 % der Theorie
$n_D^{30}$ : 1,5707

Analog zu diesem Beispiel wurden auch die folgenden erfindungsgemäßen Verbindungen hergestellt:

| Beispiel Nr. | $R^1$ | X | Y | Z | n | m | Physikalische Konstante |
|---|---|---|---|---|---|---|---|
| 2 | $-(CH_2)_2CN$ | F | Cl | 0 | 2 | 0 | Fp.: 93°C |
| 3 | $-(CH_2)_2Cl$ | F | Cl | 0 | 2 | 0 | Fp.: 88°C |
| 4 | $-CH(CH_3)_2$ | F | Cl | 0 | 2 | 0 | Fp.: 123°C |
| 5 | $-(CH_2)_4CH_3$ | F | Cl | 0 | 2 | 0 | $n_D^{20}$: 1,57390 |
| 6 | $-CH_2-CH=CH_2$ | F | Cl | 0 | 2 | 0 | Fp.: 59°C |
| 7 | $-CH_2-$ 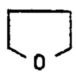 | F | Cl | 0 | 2 | 0 | $n_D^{20}$: 1,58262 |
| 8 | - 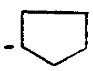 | F | Cl | 0 | 2 | 0 | Fp.: 114°C |
| 9 | $-CH_2C\equiv CH$ | F | Cl | 0 | 2 | 0 | Fp.: 102°C |

| Beispiel Nr. | $R^1$ | X | Y | Z | n | m | Physikalische Konstante |
|---|---|---|---|---|---|---|---|
| 10 | $-CH_3$ | F | Cl | 0 | 2 | 0 | Fp.: 129°C |
| 11 | $-CH_2CO_2CH_3$ | F | Cl | 0 | 2 | 0 | Fp.: 80°C |
| 12 | $-CH_2CO_2CH(CH_3)_2$ | F | Cl | 0 | 2 | 0 | Fp.: 76°C |
| 13 | $-CH_2CO_2CH_2C\equiv CH$ | F | Cl | 0 | 2 | 0 | Fp.: 81-83°C |
| 14 | $-CH_2CO_2C_5H_{11}$ | F | Cl | 0 | 2 | 0 | $n_D^{20}$: 1,5571 |
| 15 | $-CH_2CO_2-$ 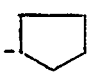 | F | Cl | 0 | 2 | 0 | $n_D^{20}$: 1,5594 |
| 16 | $-CHF_2$ | F | Cl | 0 | 2 | 0 | Fp.: 118°C |

Die folgenden Beispiele erläutern die Anwendungsmöglichkeiten der erfindungsgemäßen Verbindung:

Beispiel A

Im Gewächshaus wurden die aufgeführten Pflanzenspezies nach dem Auflaufen mit den aufgeführten Verbindungen in einer Aufwandmenge von 0,1 kg Wirkstoff/ha behandelt. Die Verbindungen wurden zu diesem Zweck als Emulsionen oder Suspensionen mit 500 Litern Wasser/ha gleichmäßig über die Pflanzen versprüht. Hier zeigten 3 Wochen nach der Behandlung die erfindungsgemäßen Verbindungen eine hohe Kulturpflanzenselektivität bei ausgezeichneter Wirkung gegen das Unkraut. Das Vergleichsmittel zeigte nicht die hohe Effektivität.

In der folgenden Tabelle bedeuten:

0 = nicht geschädigt
4 = total vernichtet.
TRZAX = Triticum aestivum
ZEAMX = Zea mays
HELAN = Helianthus annuus
SORHA = Sorghum halepense
ABUTH = Abutilon theophrasti
GALAP = Galium aparine
IPOSS = Ipomoea purpurea
MATCH = Matricaria chamomilla
POLSS = Polygonum sp.
SEBEX = Sesbania exaltata
SOLSS = Solanum sp.
VERPE = Veronica persica
VIOSS = Viola sp.

| Erfindungsgemäße Verbindungen | TRZAX | ZEAMX | HELAN | SORHA | ABUTH | GALAP | IPOSS | MATCH | POLSS | SEBEX | SOLSS | VERPE | VIOSS |
|---|---|---|---|---|---|---|---|---|---|---|---|---|---|
| Beispiel 1 | 0 | 0 | 4 | 3 | 4 | 4 | 4 | 3 | 4 | 4 | 4 | 4 | 4 |
| Beispiel 2 | 2 | 1 | 3 | 3 | 4 | 3 | 4 | 2 | 1 | 3 | 4 | 4 | 2 |
| Beispiel 6 | 2 | 1 | 2 | 3 | 4 | 4 | 3 | 3 | 3 | 4 | 4 | 4 | 3 |
| Beispiel 7 | 1 | 1 | 2 | 2 | 1 | 3 | 1 | 3 | 3 | 4 | 4 | 4 | 3 |
| Beispiel 11 | 1 | 1 | 4 | 2 | 4 | 3 | 4 | 3 | 3 | 4 | 4 | 4 | 4 |
| Beispiel 12 | 0 | 2 | 4 | 1 | 4 | 2 | 4 | 3 | 2 | 3 | 4 | 3 | 2 |
| Beispiel 13 | 0 | 0 | 4 | 1 | 4 | 2 | 4 | 2 | 2 | 4 | 4 | 4 | 3 |
| Beispiel 14 | 1 | 1 | 4 | 1 | 4 | 3 | 4 | 3 | 2 | 4 | 4 | 3 | 3 |
| Beispiel 15 | 1 | 0 | 4 | 2 | 4 | 3 | 4 | 3 | 3 | 3 | 4 | 4 | 3 |
| Unbehandelt | 0 | 0 | 0 | 0 | 0 | 0 | 0 | 0 | 0 | 0 | 0 | 0 | 0 |
| Vergleichsmittel | | | | | | | | | | | | | |
| Oxadiazon | 0 | 1 | 1 | 2 | 4 | 2 | 3 | 2 | 3 | 2 | 3 | 3 | 4 |

Beispiel B

Im Gewächshaus wurden die aufgeführten Pflanzenspezies vor dem Auflaufen mit den aufgeführten Verbindungen in einer Aufwandmenge von 0,1 kg Wirkstoff/ha behandelt. Die Verbindungen wurden zu diesem Zweck als Emulsionen oder Suspensionen mit 500 Litern Wasser/ha gleichmäßig über die Pflanzen versprüht. Hier zeigten 3 Wochen nach der Behandlung die erfindungsgemäße Verbindung eine hohe Kulturpflanzenselektivität in Soja (Glyzine max/GLXMA), Weizen (Triticum aestivum/TRZAX) und Mais (Zea mays/ZEAMX) bei ausgezeichneter Wirkung gegen das Unkraut. Das Vergleichsmittel zeigte nicht die gleichhohe Wirksamkeit.

In der folgenden Tabelle bedeuten:
0 = nicht geschädigt
4 = total vernichtet.
GLXMA = Glycine max
TRZAX = Triticum aestivum
ZEAMX = Zea mays
ABUTH = Abutilon theophrasti
GALAP = Galium aparine
IPOSS = Ipomoea purpurea
MATCH = Matricaria chamomilla
SEBEX = Sesbania exaltata
SOLSS = Solanum sp.
VERPE = Veronica persica

| Erfindungsgemäße Verbindung | GLXMA | TRZAX | ZEAMX | ABUTH | GALAP | IPOSS | MATCH | SEBEX | SOLSS | VERPE |
|---|---|---|---|---|---|---|---|---|---|---|
| Beispiel 1 | 0 | 0 | 0 | 4 | 4 | 3 | 4 | 4 | 4 | 3 |
| Unbehandelt | 0 | 0 | 0 | 0 | 0 | 0 | 0 | 0 | 0 | 0 |
| Vergleichsmittel | | | | | | | | | | |
| Oxadiazon | 0 | 0 | 1 | 2 | 2 | 3 | 3 | 2 | 4 | 2 |

Beispiel C

Im Gewächshaus wurden die in der Tabelle aufgeführten Verbindungen mit den ebenfalls erwähnten Aufwandmengen appliziert. Hierzu wurden die Wirkstoffe in Gefäße mit 1500 ml Wasser gegeben. Als Testpflanzenarten wurden Echinochloa crus-galli (ECHCG). Cyperus esculentus (CYPES), Cyperus difformis (CYPDI) und Sirpus juncoides (SCPJU) im 2- bis 5-Blatt-Stadium eingesetzt. Drei Wochen nach der Applikation wurde die Schädigung der Pflanzen bonitiert, und zwar nach folgendem Schema:
0 = keine Schädigung
1 = schwache Schädigung
2 = mittlere Schädigung
3 = starke Schädigung
4 = total vernichtet.
\- = nicht geprüft

| Erfindungsgemäße Verbindung | Wasserapplikation ppm | ECHCG | CYPES | CYPDI | SCPJU |
|---|---|---|---|---|---|
| Beispiel 1 | 10 | 4 | 4 | 4 | 3 |
| Beispiel 11 | 10 | 4 | - | - | - |
| Beispiel 12 | 10 | 4 | - | - | - |
| Beispiel 13 | 10 | 4 | - | - | - |
| Beispiel 14 | 10 | 4 | - | - | - |

Wie die Tabelle zeigt, sind die erfindungsgemäßen Verbindungen stark wirksam gegen wichtige Reisunkräuter.

Beispiel D

Im Gewächshaus wurden die aufgeführten Pflanzenspezies vor dem Auflaufen mit den aufgeführten Verbindungen in einer Aufwandmenge von 0,3 kg Wirkstoff/ha behandelt. Die Verbindungen wurden zu diesem Zweck als Emulsionen oder Suspensionen mit 500 Litern Wasser/ha gleichmäßig über die Pflanzen versprüht. Hier zeigt drei Wochen nach der Behandlung die Thio-Verbindung eine hohe Kulturpflanzenselektivität in Mais (ZEAMX) bei ausgezeichneter Wirkung gegen das Unkraut. Die entsprechende Sauerstoff-Verbindung zeigte nicht die gleichhohe Wirksamkeit.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung
ZEAMX = Zea mays
SORHA = Sorghum halepense
ABUTH = Abutilon theophrasti
GALAP = Galium aparine
MATCH = Matricaria chamomilla
SOLSS = Solanum sp.
VERPE = Veronica persica

| Erfindungsgemäße Verbindung | ZEAMX | SORHA | ABUTH | GALAP | MATCH | SOLSS | VERPE |
|---|---|---|---|---|---|---|---|
| Beispiel 1 | 0 | 3 | 4 | 4 | 4 | 4 | 3 |
| Vergleichsmittel (gemäß DE-OS 2 801 429) | | | | | | | |
| 2-(4-Chlor-5-ethoxycarbonylmethoxy-2-fluorphenyl)-5,6,7,8-tetrahydro-1,2,4-triazolo[4,3-a]-pyridin-3(2H)-on | 0 | 0 | 0 | 2 | 3 | 2 | 0 |

## Beispiel E

Im Gewächshaus wurden die aufgeführten Pflanzenspezies nach dem Auflaufen mit den aufgeführten Verbindungen in einer Aufwandmenge von 0,03 kg Wirkstoff/ha behandelt. Die Verbindungen wurden zu diesem Zweck als Emulsionen oder Suspensionen mit 500 Litern Wasser/ha gleichmäßig über die Pflanzen versprüht. Hier zeigt zwei Wochen nach der Behandlung die Thio-Verbindung eine hohe Kulturpflanzenselektivität in Mai (ZEAMX) bei ausgezeichneter Wirkung gegen das Unkraut. Die entsprechende Sauerstoff-Verbindung zeigte nicht die gleichhohe Wirksamkeit.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung
ZEAMX = Zea mays
ABUTH = Abutilon theophrasti
GALAP = Galium aparine
IPOSS = Ipomoea purpurea
MATCH = Matricaria chamomilla
SOLSS = Solanum sp.
VERPE = Veronica persica
VIOSS = Viola sp.

| Erfindungsgemäße Verbindung | ZEAMX | ABUTH | GALAP | IPOSS | MATCH | SOISS | VERPE | VIOSS |
|---|---|---|---|---|---|---|---|---|
| Beispiel 1 | 0 | 4 | 3 | 4 | 4 | 4 | 3 | 3 |
| Vergleichsmittel (gemäß DE-OS 2 801 429) | | | | | | | | |
| 2-(4-Chlor-5-ethoxycarbonylmethoxy-2-fluorphenyl)-5,6,7,8-tetrahydro-1,2,4-triazolo[4,3-a]-pyridin-3(2H)-on | 0 | 0 | 0 | 0 | 0 | 0 | 0 | 0 |

## Ansprüche

1. Substituierte bicyclische Triazole der allgemeinen Formel I

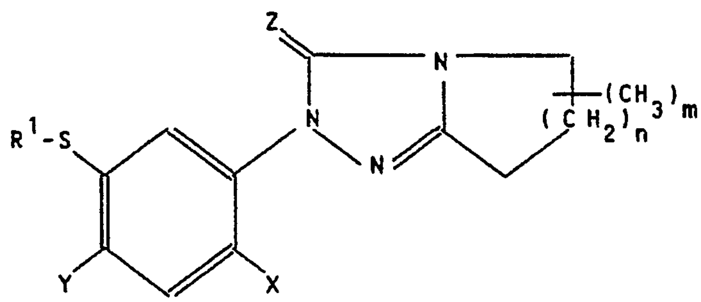

(I) ,

in der
X ein Wasserstoffatom oder ein Fluoratom,
Y ein Halogenatom,
Z ein Sauerstoffatom oder ein Schwefelatom,
$R^1$ einen $C_1$-$C_6$-Alkyl, $C_2$-$C_6$-Alkenyl- oder $C_3$-$C_6$-Alkinylrest, einen ein-oder mehrfach durch Halogen und/oder $C_1$-$C_4$-Alkoxy substituierten $C_1$-$C_6$-Alkyl-, $C_2$-$C_6$-Alkenyl- oder $C_3$-$C_6$-Alkinylrest, einen $C_3$-$C_7$-Cycloalkyl- oder $C_5$-$C_7$-Cycloalkenylrest, bei dem gegebenenfalls ein oder zwei Kohlenstoffatome durch Sauerstoffatome ersetzt sind, einen ein-oder mehrfach durch Halogen und/oder $C_1$-$C_4$-Aloxy substituierten $C_3$-$C_7$-Cycloalkyl- oder $C_5$-$C_7$-Cycloalkenylrest, bei dem gegebenenfalls ein oder zwei Kohlenstoffatome durch Sauerstoffatome ersetzt sind, einen $C_3$-$C_7$-Cycloalkyl-$C_1$-$C_3$-alkyl- oder $C_5$-$C_7$-Cycloalkenyl-$C_1$-$C_3$-alkylrest, bei dem gegebenenfalls ein oder zwei Kohlenstoffatome durch Sauerstoffatome ersetzt sind, einen ein- oder mehrfach durch Halogen und/oder $C_1$-$C_4$-Alkoxy substituierten $C_3$-$C_7$-Cycloalkyl-$C_1$-$C_3$-alkyl-oder $C_5$-$C_7$-Cycloalkenyl-$C_1$-$C_3$-alkylrest, bei dem gegebenenfalls ein oder zwei Kohlenstoffatome durch Sauerstoffatome ersetzt sind, einen Cyano-$C_1$-$C_3$-alkylrest oder die Gruppe $CR^2R^3A$,
$R^2$ ein Wasserstoffatom, ein Fluoratom oder einen Methylrest,
$R^3$ ein Wasserstoffatom, ein Fluoratom oder einen Methylrest,
A eine der Gruppen $CO_2R^4$, $COSR^5$ oder $CONR^6R^7$,

$R^4$ einen $C_1$-$C_6$-Alkyl-, $C_2$-$C_6$-Alkenyl- oder $C_3$-$C_6$-Alkinylrest, einen ein-oder mehrfach durch Halogen und/oder $C_1$-$C_4$-Alkoxy substituierten $C_1$-$C_6$-Alkyl-, $C_2$-$C_6$-Alkenyl- oder $C_3$-$C_6$-Alkinylrest, eine $C_3$-$C_7$-Cycloalkyl- oder $C_5$-$C_7$-cycloalkenylrest, bei dem gegebenenfalls ein oder zwei Kohlenstoffatome durch Sauerstoffatome ersetzt sind, einen ein-oder mehrfach durch Halogen und/oder $C_1$-$C_4$-Alkoxy substituierten $C_3$- $C_7$-Cycloalkyl- oder $C_5$-$C_7$-Cycloalkenylrest, bei dem gegebenenfalls ein oder zwei Kohlenstoffatome durch Sauerstoffatome ersetzt sind, einen $C_3$-$C_7$-Cycloalkyl-$C_1$-$C_3$-alkyl- oder $C_5$-$C_7$-Cycloalkenyl-$C_1$-$C_3$-alkylrest, bei dem gegebenenfalls ein oder zwei Kohlenstoffatome durch Sauerstoffatome ersetzt sind, einen ein- oder mehrfach durch Halogen und/oder $C_1$-$C_4$-Alkoxy substituierten $C_3$-$C_7$-Cycloalkyl-$C_1$-$C_3$-alkyl-oder $C_5$-$C_7$-Cycloalkenyl-$C_1$-$C_3$-alkylrest, bei dem gegebenenfalls ein oder zwei Kohlenstoffatome durch Sauerstoffatome ersetzt sind, oder einen Cyano-$C_1$-$C_3$-alkylrest,

$R^5$ einen $C_1$-$C_6$-Alkyl-, $C_2$-$C_6$-Alkenyl- oder $C_3$-$C_6$-Alkinylrest oder einen ein- oder mehrfach durch Halogen und/oder $C_1$-$C_4$-Alkoxy substituierten $C_1$-$C_6$-Alkyl-, $C_2$-$C_6$-Alkenyl- oder $C_3$-$C_6$-Alkinylrest,

$R^6$ ein Wasserstoffatom, einen $C_1$-$C_6$-Alkyl-, $C_2$-$C_6$-Alkenyl- oder $C_3$-$C_6$-Alkinylrest oder einen Phenylrest,

$R^7$ ein Wasserstoffatom, einen $C_1$-$C_6$-Alkyl-, $C_2$-$C_6$-Alkenyl- oder $C_3$-$C_6$-Alkinylrest oder einen Phenylrest oder

$R^6$ und $R^7$ gemeinsam mit dem benachbarten Stickstoffatom die Morpholino-, Piperidino- oder Pyrrolidinylgruppe,

n die Zahlen 1, 2 oder 3 und

m die Zahlen 0, 1, 2 oder 3

bedeuten.

2. 2-(4-Chlor-5-ethoxycarbonylmethylthio-2-fluorphenyl)-5,6,7,8-tetrahydro-1,2,4-triazole[4,3-a]pyridin-3-(2H)-on

3. Verfahren zur Herstellung von substituierten bicyclischen Triazolen der allgemeinen Formel I gemäß den Ansprüchen 1 und 2, dadurch gekennzeichnet, daß man

A) falls Z für Sauerstoff steht, eine Verbindung der allgemeinen Formel II

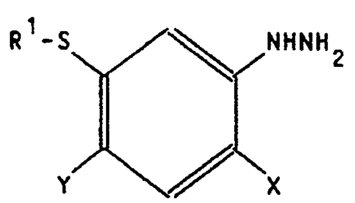

(II) ,

in der X, Y und $R^1$ die unter der allgemeinen Formel I genannten Bedeutungen haben, mit einer Verbindung der allgemeinen Formel III

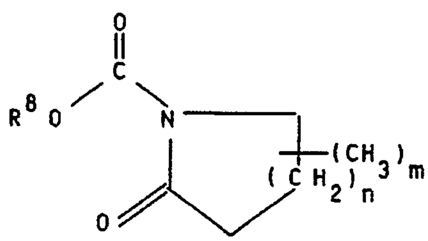

(III) ,

in der n und m die unter der allgemeinen Formel I genannte Bedeutung haben und $R^8$ für $C_1$-$C_4$-Alkyl steht, in einem inerten Lösungsmittel in Gegenwart von Phosphorpentoxid umsetzt, oder

B) eine Verbindung der allgemeinen Formel IV

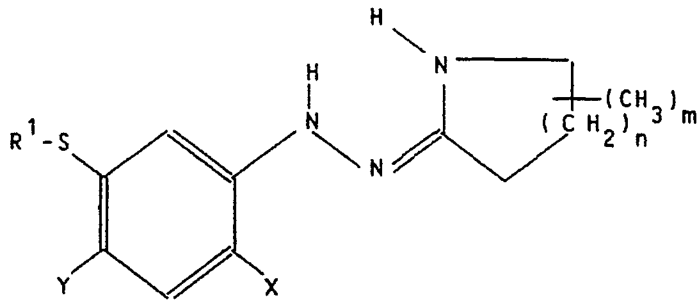

(IV) ,

in der X, Y, $R^1$, n und m die unter der allgemeinen Formel I genannten Bedeutungen haben, gegebenenfalls als Salz mit einer Verbindung der allgemeinen Formel V

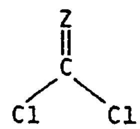

(V) ,

in der Z die unter der allgemeinen Formel I genannte Bedeutung hat, in einem inerten Lösungsmittel in Gegenwart einer Base umsetzt.

4. Herbizide Mittel, gekennzeichnet durch einen Gehalt an mindestens einer Verbindung gemäß den Ansprüchen 1 und 2.

5. Verwendung von Mitteln gemäß dem Anspruch 4 zur Bekämpfung monokotyler und dikotyler Unkrautarten in landwirtschaftlichen Hauptkulturen.

6. Verfahren zur Herstellung von herbiziden Mitteln, dadurch gekennzeichnet, daß man Verbindungen der allgemeinen Formel I gemäß den Ansprüchen 1 oder 2 mit Träger- und/oder Hilfsstoffen vermischt.